Europäisches Patentamt

European Patent Office

Office européen des brevets

(19)

(11) Publication number: **0 022 765**
**B1**

(12) **EUROPEAN PATENT SPECIFICATION**

(45) Date of publication of patent specification: **10.08.83**

(51) Int. Cl.³: **B 01 L 3/14, B 65 D 41/18**

(21) Application number: **78900298.7**

(22) Date of filing: **28.11.78**

(86) International application number:
**PCT/EP78/00023**

(87) International publication number:
**WO 80/01047 29.05.80 Gazette 80/12**

(54) **TUBE AND STOPPER FOR BLOOD-SAMPLING SYSTEMS.**

(43) Date of publication of application:
**28.01.81 Bulletin 81/4**

(45) Publication of the grant of the patent:
**10.08.83 Bulletin 83/32**

(84) Designated Contracting States:
**CH DE FR GB SE**

(56) References cited:
**BE - A - 497 328**
**DE - A - 2 355 511**
**FR - A - 1 199 247**
**FR - A - 1 552 277**
**FR - A - 1 583 447**
**FR - A - 1 586 053**
**FR - A - 2 040 666**
**FR - A - 2 071 306**
**GB - A - 822 756**
**US - A - 2 739 564**
**US - A - 3 005 564**
**US - A - 3 615 222**
**US - A - 3 921 845**
**US - A - 4 066 067**

(73) Proprietor: **DEMATEX DEVELOPMENT &**
**INVESTMENT ESTABLISHMENT**
**FL-9490 Vaduz (LI)**

(72) Inventor: **RILLIET, François**
**Route J.J. Rigand, 18A**
**1224 Chêne-Bougeries, Genève (CH)**
Inventor: **MICHELI, Antoine**
**Landecy**
**1257 La Croix de Rozon, Genève (CH)**

(74) Representative: **Jörchel, Dietrich R.A.**
**c/o BUGNION S.A. Conseils en Propriété**
**Industrielle 10, route de Florissant Case postale**
**375**
**CH-1211 Genève 12 Champel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Tube and stopper for blood-sampling systems

Technical field

The invention relates to stoppers for vial-type tubes, in particular tubes subjected to a controlled pressure, for example evacuated tubes of blood sampling systems.

Background art

A known blood sampling system comprises an evacuated tube closed by a stopper and an open-ended needle-holder tube slidably mounted on the tube or its stopper. This needle holder carries a hollow needle having one part protruding axially from the closed end of the holder for pricking into a vein, and another part extending axially within the holder, the latter part being encased in a loose, flexible cover or sleeve. To take a blood sample, the needle is pricked into a vein and the evacuated tube displaced until the needle pierces the stopper membrane, so that blood is sucked into the evacuated tube.

However, in practice conventional blood sampling systems are often difficult to use and have shortcomings that create health hazard conditions for laboratory staff and may alter the results of some blood determinations.

Conventional stoppers usually have a shank which penetrates inside the tube neck, the shank having an externally bevelled end which is hollow to facilitate entry into the tube. As a consequence, blood particles may aggregate in recesses in the shank and when, after centrifugation, the stopper is removed these trapped particles may fall down into the centrifuged sample and alter the result of certain blood determinations.

Also, when the stopper is removed, blood particles on the shank tend to be sprayed as an aerosol, as a result of the "bouncing" effect. This is one of the main ways in which virus or bacteria contaminate laboratory staff. Handling such a stopper which has a surface smeared with blood is thus not only an unpleasant operation, but involves health hazards.

Many problems inherent to traditional evacuated tubes stem from the fact that sealing is achieved solely by the pressure of the outside wall of the stopper against the inner surface of the tube. This single sealing means serves both to prevent escape of the liquid content (blood) and penetration of air from the exterior. Removing or inserting the stopper produces instantaneous drafts of air which contribute to the aerosol effect.

Moreover, once such a stopper has been removed from an evacuated tube and the vacuum is lost, when it is reinserted in the tube it tends to be ejected as a result of compression of air in the tube. It is thus not suitable for reinsertion as a permanent closure.

A known arrangement which avoids some of these drawbacks has a stopper with a skirt fitting over the tube neck, the inner surface of this skirt having intersecting axially-directed and annular grooves which cooperate with an external bead on the tube neck to provide a venting system (see Figs. 13 and 14 of U.S. Patent No. 4,066,067. With this arrangement, however, sealing was provided only on the outside wall of the tube by a single sealing means, essentially by the tight fit of the bead in an annular groove of deeper section than the axially-directed groove.

Disclosure of the invention

The invention concerns the combination of a vial-type tube and a stopper, the tube comprising a neck having an open end. The stopper comprises a hollow generally cylindrical body of deformable material having a head including a sealing membrane for fitting over and closing the open end of the tube and an integral skirt extending from the head for sealably fitting over the neck of the tube. In the stopper, there is at least one axially-directed groove extending from the edge of the skirt at least partly along the inner surface of the skirt. The stopper is movable outwardly on the tube from a sealing position to a venting position in which the axially-directed groove communicates the interior of the tube with the exterior.

An object of the invention is to provide such a combination with an improved dual-sealing means, the combination according to the invention being characterized in that the stopper has an annular recess surrounding a protruding central plug of the sealing membrane that sealably fits in the open end of the tube neck when the stopper is in the sealing position and in that the skirt has a generally cylindrical sealing portion which extends from said annular recess beyond the plug and is smooth and uninterrupted until it reaches said axially-directed groove, said sealing portion having a smaller diameter than the maximum diameter of the annular recess such that in the sealing position it sealably fits around the outer wall of the tube neck when simultaneously the plug sealably fits against the inner wall of the tube neck.

Only a limited and generally inaccessible part of the stopper, namely the central plug of the sealing membrane—which usually has a flat surface—can contact blood in the tube. This plug downwardly protruding from the membrane acts as a tight sealing means for the liquid content. The inner wall of the skirt, which assumes the main part of the vacuum retention function, is protected from any seepage of the tube content and stays dry. Thus, the sealing function is provided by two separate means, the plug for liquids (blood), and the inner skirt wall for air. In most instances, i.e. especially for blood collection tubes with a thin-walled neck, to provide a dual sealing arrangement in which the central plug fits tightly against the inner wall of the tube neck and the inner skirt wall fits tightly against the outer wall of the tube neck, the stopper will be so dimensioned that when it is in its unstressed state, i.e. removed from the tube, the outer diameter of the plug will be equal to or larger than the inner diameter of the skirt at least in the sealing portion of the skirt. This is made feasible by making said annular recess in the form of an inwardly-facing annular groove in the end part of the skirt surrounding the plug, this groove extending beyond the plug to the generally cylindrical sealing portion of the skirt which is smooth and uninterrupted.

Also, in this dual-sealing system, the primary purpose of the plug is to provide liquid tight sealing which does not require such high radially-acting pressures as for vacuum preservation. The plug can therefore advantageously be made with a plain configuration and a flat bottom as opposed to the hollow, highly deformable structures of conventional stopper shanks forming a single sealing means. Thus the plug will have no unoccupied volumes or recesses in which blood can form deposits. Nevertheless, other forms of stopper could be made in which each of two sealing means may serve for both sealing functions (liquid content and air penetration from the exterior).

All the outside surface of the stopper, as well as the inside wall of the skirt, is totally isolated from the tube content and stays dry and clean. The fitting-over form of the skirt incorporating a dual sealing system and a venting system hence makes the stopper convenient to handle and safe.

The venting system eliminates the aforementioned bouncing effect when the stopper is removed, by balancing pressures before the stopper is removed. Removal of the stopper is thus easy and smooth and the spraying of blood particles in an aerosol is avoided. The venting system can also be used to facilitate initial fitting of the stopper and evacuation of the tube, by carrying out evacuation with the stopper already placed on the tube in the venting position.

In one embodiment, the tube neck has an outwardly protruding annular bead on the end of its neck and the stopper skirt has at least one annular groove (i.e. including the annular recess surrounding the plug) configured in cross-section to receive therein this annular bead with a sealing fit. In this embodiment the stopper is, in the same way, movable outwardly, in relation to the tube, from a sealing position, in which the bead fits sealably in said annular recess, to a venting position in which the axially-directed groove communicates the interior of the tube with the exterior.

The complementary configurations of the bead on the tube and the annular recess or groove in the stopper enable the stopper to be reseated on the tube as a permanent closure after loss of vacuum. This is further facilitated by the venting system, and by the fact that, at most, the stopper plug can only slightly penetrate in the tube, so that air in the tube will not be appreciably compressed.

In use, after a blood sample has been sucked into the evacuated tube, aspiration of the sample from the tube is facilitated by placing the stopper in the venting position, i.e. without a need to remove the stopper and open the tube.

The fitting-over form of the stopper skirt also enables the stoppering of vials and tubes having a neck of a smaller diameter than is suitable for receiving a conventional plug-like stopper.

For analyses in which blood samples must be kept vented with sterile air, the axial groove will be occupied by an aseptized gas-permeable filter material adhering to its surface and fitting tightly against the outer wall of the tube neck when the stopper is held on the tube in venting position so that gaseous exchanges between the interior of the tube and the atmosphere are channelled through this aseptized filter material.

Brief description of drawings

Embodiments of the invention will now be described, by way of example, with reference to the accompanying schematic drawings, in which:

Fig. 1 is a cross-section of a first form of stopper fitted on a tube in a pushed-in sealing position, the same stopper and tube being shown separated from one another in Fig. 2;

Fig. 3 shows a variation of the embodiment of Fig. 1 in which the tube has an inwardly-directed rim;

Fig. 4 is a cross-section of a second form of stopper fitted on a tube, in a pushed-in sealing position;

Fig. 5 shows the stopper and tube of Fig. 4, in a pulled-out venting position;

Fig. 6 is a view similar to Fig. 5 with a modified stopper;

Fig. 7 is a cross-section through the skirt of the stopper of Fig. 6;

Fig. 8 shows another varied form of the stopper of Figs. 4 and 5 in cross-section, and the neck of a tube in elevation; and

Fig. 9 is a cross-section of part of a stopper similar to that of Figs. 4 and 5 fitted on a modified tube, in a sealing position.

Best modes for carrying out the invention

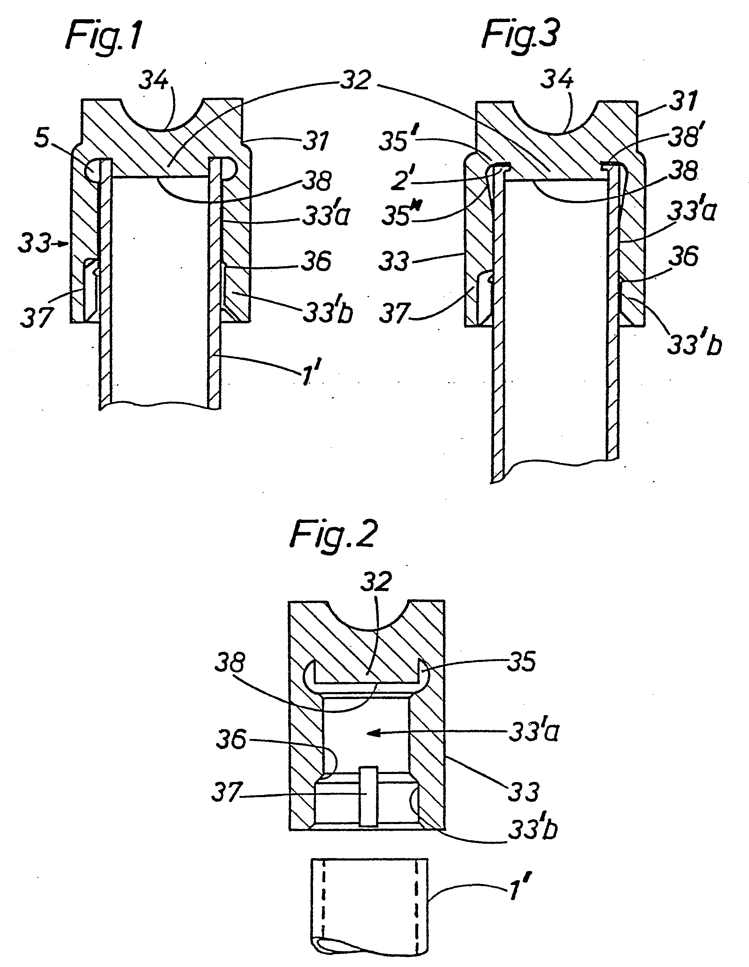

Fig. 1 shows a tube and stopper combination of which the tube neck 1′ has a smooth cylindrical outer surface, and the stopper consists of a body of deformable material, such as synthetic rubber, having a head 31 and an integral skirt 33 extending flush from the generally cylindrical side wall of the head. A self-sealing membrane 32 is defined by a central dimple 34 in the head 31. The membrane 32 includes a central plug 38 surrounded by an annular recess in the form of an inwardly-directed groove 35 in the end of skirt 33 adjacent head 31. The inner wall of the skirt 33 is divided into two cylindrical portions, an upper one 33′*a* extending to the groove 35 and a lower one 33′*b* extending to the skirt edge. The lower portion 33′*b* has a slightly larger diameter than the upper one 33′*a*, the two portions being connected by a slanted section 36 (shown in Fig. 2). The inner wall of the skirt 33 also has an axially-directed groove 37 which extends from the skirt edge along the lower portion 33′*b* and slightly into the upper portion 33′*a* which, otherwise, is smooth and uninterrupted.

Prior to tube introduction, that is when the skirt 33 is unstressed, the inside diameter of portion 33′*b* is smaller than the outside diameter of the tube neck 1′. When the tube neck 1′ is introduced into the lower portion 33′*b* of the stopper skirt the upper cylindrical portion 33′*a* remains inset compared to the portion 33′*b* so that the slanting section 36 forms a stop against which the external rim of the tube rests to define a venting position in which the axially-directed groove 37 communicates the interior of the tube with the exterior. When the stopper is fully pushed on by applying a further force, the upper skirt portion 33′*a* also deforms outwardly to receive the tube neck with a tight sealing fit.

Furthermore, the diameter of plug 38 is larger than the inner diameter of the tube neck so as to provide liquid tight sealing when the stopper is fully pushed in, as shown in Fig. 1. Owing to the thinness of the tube wall, in most instances the diameter of plug 38 will be practically equal or even larger than the diameter of the upper portion 33′*a* in the unstressed state of the skirt, so that in the pushed-in position this portion 33′*a* will provide air tight sealing against the outer surface of the tube when simultaneously the plug 38 provides liquid-tight sealing against the inner surface of the tube. This dual-sealing arrangement is made feasible by the combination of three elements: the annular groove 35, the central plug 38 and the upper portion 33′*a* of the skirt (i.e. its uninterrupted effective sealing portion above the end of groove 37). In this combination, the annular groove 35 inwardly facing the plug 38 but extending beyond it provides a discontinuity or open space between the two other elements, the plug 38 and the upper portion 33′*a*, in which the thin tube wall is received and clamped. Hence the upper annular groove 35 is an important element of the dual sealing system, even though the neck of the tube has a straight configuration, i.e. has no external annular bead to lodge.

It would be possible, however, by resorting to an unusually thick tube wall, to make the outer diameter of the plug slightly smaller than the inner diameter of the skirt when the stopper is unstressed.

Fig. 3 shows a variation of the tube and stopper combination of Fig. 1. The tube 1′ has a rim 2′, slightly protruding as an inward bead, at the end of the neck. The stopper has an outwardly facing annular groove 38′, insert in the periphery of plug 38 for sealably engaging with the rim 2′. Sealing is further improved by the sealing surface portion 33′*a* of the skirt 33 which tightly fits about the tube neck. The annular recess 35 is interrupted at its maximum diameter 35′, the inner surface of the skirt 33 having a generally conical section 35″ flaring outwardly from the sealing portion 33′*a* to 35′. All the other parts are unchanged and designated by the same references as in Fig. 1. The slanting section 36 may, if desired, be an annular groove.

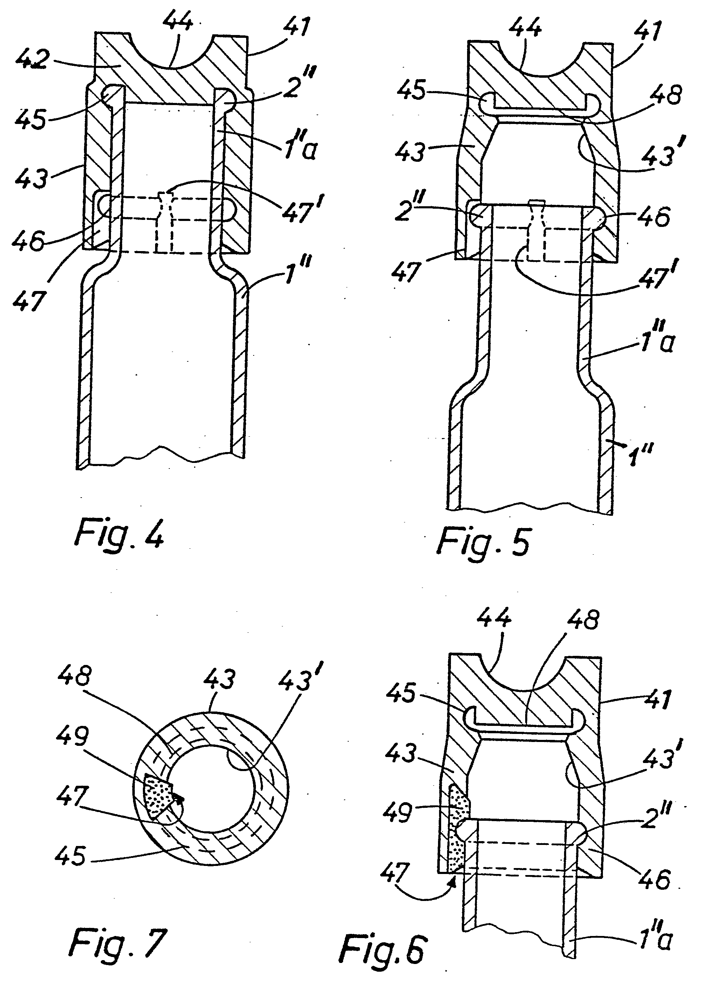

Figs. 4 and 5 show a tube and stopper combination of which the stopper consists of a head 41 and an integral skirt 43 extending flush from the generally cylindrical side wall of the head. A sealing membrane 42 is defined by a central dimple 44 in the head 41. Membrane 42 includes a central inwardly-protruding plug 48 which is surrounded by a first inwardly-facing annular groove 45 in the end part of the skirt 43, adjacent head 41. A second annular groove 46 is provided in the skirt near its edge and axially-directed grooves 47, 47′ extend from the edge of skirt 43, partly along the inner surface of the skirt which has a smooth uninterrupted surface portion 43′ extending to the groove 45. As shown, the axially-directed grooves 47, 47′ are deeper than and intersect the annular groove 46. Also, when the stopper is unstressed, the sealing portion 43′ of the skirt has a smaller diameter than the plug 48 (see Fig. 5).

The annular grooves 45, 46 are each shaped in cross-section to receive an outwardly-projecting annular bead 2″ forming a rim on the open end of the cylindrical neck 1″*a* of vial-like tube 1″. The skirt 43 has approximately the same length as the neck 1″*a* and an outer diameter such that when the stopper is fully pushed on the tube neck 1″*a*, i.e. with the bead 2″ located in groove 45, it is substantially flush with the enlarged cylindrical body of tube 1″. In this position the bead 2″ sealably fits in groove 45, and the portion 43′ of the skirt closely circumferentially fits on the neck 1″*a*. Also, the plug 48 penetrates slightly into the open end of neck 1″*a* to improve sealing especially preventing the escape of any liquid content of tube 1″. When the stopper is pulled out until the bead 2″ engages in groove 46, the axially-directed grooves 47, 47′ communicate the inside of tube 1″ with the exterior. The stopper/tube combination thus has discrete sealing and venting positions.

During blood sampling, the stopper of course remains in the sealing position. However, to remove the blood sample after centrifugation, the stopper is pulled out to the venting position. This allows air to enter the tube and then, by piercing the membrane 42 with a hollow probe or other pipetting device connected to an evacuating pump, the blood sample can be pipetted out of the tube without removing the stopper. Hence, processing of the tube sample, including pipetting out the content, can be done with the tube stoppered.

Handling of the tube/stopper assembly is facilitated and, even when the stopper is removed, is clean and hygienic since the blood can only contact the flat inner surface of plug 48 which is shielded by the skirt 43. As with the other embodiments, at no time does the inside of the skirt or its outer peripheral surface come into contact with the tube content.

Figs. 6 and 7 show an adaptation of the stopper of Figs. 4 and 5 having a single axially-directed groove 47 of somewhat larger dimensions than before, this groove 47 having a dovetail cross-section and being filled with an asepticized gas-permeable filtering material 49. When the stopper is unstressed (Fig. 7), the material 49 protrudes inwardly from the sealing surface portion 43' of the stopper skirt 43 so that when the stopper is placed in the venting position (Fig. 6) the material 49 is tightly pressed against the outer wall of the tube neck. The material 49 will preferably have an indent corresponding to the annular groove 46 so as to avoid undue compression by the bead 2''.

Fig. 7 also shows, in dotted lines, the diameter of the plug 48 which is greater than the diameter of the sealing portion 43' in the unstressed state, and the outer diameter of groove 45 which is even greater.

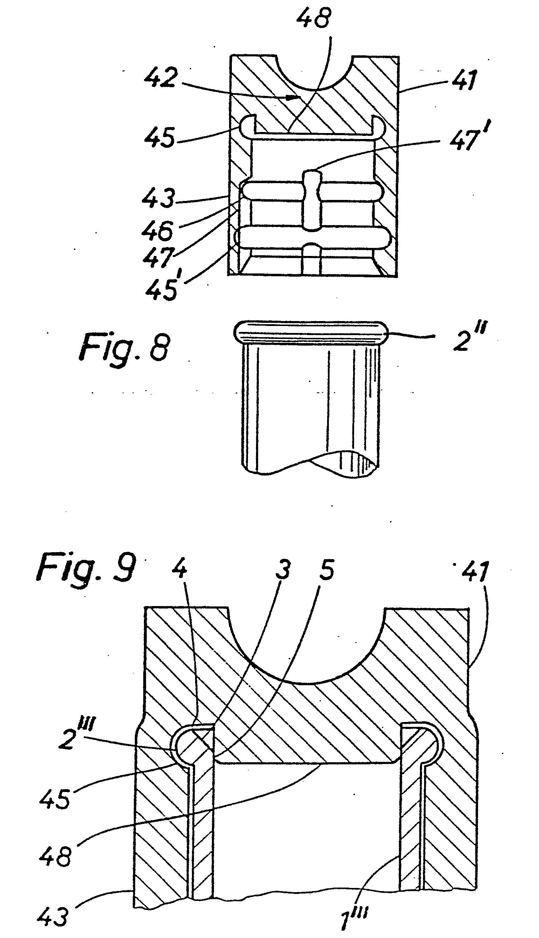

Fig. 8 shows a modified form of the stopper of Figs. 4, 5 having a third annular groove 45' spaced apart from the groove 46 towards the edge of skirt 43. The axially-directed grooves 47, 47' intersect grooves 45' and 46; they are shallower than groove 45', and deeper than groove 46. Thus, with this embodiment, there is a pushed-in sealed position (bead 2'' in groove 45), an intermediate venting position (bead 2'' in groove 46) and a pulled-out sealed position (bead 2'' in groove 45').

In Fig. 9, a stopper of the type shown in Figs. 4 and 5 and identified by the same references cooperates with a tube 1''' whose cylindrical inner surface is extended, at the rim, by an outwardly-flaring trunco-conical surface 3 which terminates at the upper edge 4 of an outwardly-protruding curved external surface forming a bead 2''' on the rim. The plug 48 of stopper head 41 is dimensioned so that when the stopper is in the pushed-in sealing position with bead 2''' engaged in groove 45, the plug 48 sealably engages the cylindrical inner surface of tube 1''' at 5. In this position, a space is left between the cylindrical periphery of plug 48 and the trunco-conical surface 3. The plug 48 thus remains out of contact with the curved external surface of bead 2''' when the stopper is moved into and out of the sealing position. This avoids any blood traces being transferred from the end of the plug 48 onto the outside of the tube bead in case of repeated removal and reinsertion of the stopper. Instead of being conical, the surface 3 could for example have a concave arcuate section.

Naturally many variations may be made to the described embodiments and features of one embodiment may be combined with another embodiment, where appropriate. The term "axially-directed" groove is intended to include grooves and similar recesses having a major axial component to provide the desired venting effect, as well as equivalent venting means for communicating the interior of the tube with the exterior when the stopper is held on the tube in a venting position. Also, the stopper head could have other forms, for example a generally cylindrical hollow body having a slotted end wall for receiving the flexible sleeve of an encased needle.

**Claims**

1. In combination, a vial-type tube and stopper, the tube comprising a neck (1') having an open end and the stopper comprising a hollow generally cylindrical body of deformable material having a head (31) including a sealing membrane (32) for fitting over and closing the open end of the tube and an integral skirt (33) extending from the head (31) for fitting over the neck (1') of the tube, and at least one axially-directed groove (37) in the stopper extending from the edge of the skirt at least partly along the inner surface of said skirt, the stopper being outwardly movable on the tube from a sealing position to a venting position in which said axially-directed groove communicates the interior of the tube with the exterior, characterized in that the stopper has an annular recess (35) surrounding a protruding central plug (38) of the sealing membrane (32) that sealably fits in the open end of the tube neck when the stopper is in the sealing position, and in that the skirt has a generally cylindrical sealing portion (33'a) which extends from said annular recess (35) beyond the plug (38) and is smooth and uninterrupted until it reaches said axially-directed groove (37), said sealing portion (33'a) having a smaller diameter than the maximum diameter of the annular recess (35) such that in the sealing position it sealably fits around the outer wall of the tube neck when simultaneously the plug (38) sealably fits against the inner wall of the tube neck.

2. Tube and stopper combination according to claim 1, in which said annular recess (35) is in the form of an inwardly-facing groove in the end part of the skirt, said groove extending beyond the plug (38).

3. Tube and stopper combination according to claim 2, in which the diameter of the stopper plug (38) is greater than or equal to the diameter of the sealing portion (33'a) of the skirt when the skirt is unstressed prior to introduction of the tube neck.

4. Tube and stopper combination according to claim 2, in which the stopper (41, 43) comprises at least one further circumferential annular groove (46) in the inner surface of the skirt (43).

5. Tube and stopper combination according to claim 4, in which the or at least one of said further annular grooves intersects with said axially-directed groove (47, 47') whereby when the stopper is pulled out from the sealing position until the open end of the tube neck engages in said annular groove, the stopper is held in said venting position.

6. Tube and stopper combination according to claim 1, in which the tube neck has a smooth cylindrical outer surface and a rim (2') protruding inwardly from the open end of the neck, the stopper plug having on its peripheral surface an outwardly-facing groove (38') configured in cross-section to sealably receive therein said inwardly-protruding rim of the tube neck when the stopper is in the sealing position.

7. Tube and stopper combination according to claim 1, in which said inner surface of the skirt extends upwardly beyond the sealing portion as a generally conical section (35'') flaring outwardly from said cylindrical sealing portion (33'a) to a maximum diameter (35') at a location facing the peripheral wall of the plug.

8. Tube and stopper combination according to claim 1, in which the inner surface of the skirt comprises first and second cylindrical portions, a generally uninterrupted first sealing portion (33'a) adjacent the head of the stopper, said first sealing portion at most being interrupted only partly by an end part of said axially-directed groove (37), and a second cylindrical portion (33'b) of greater diameter than said first portion, said second portion extending from adjacent said first portion to the edge of the skirt and being interrupted by said axially-directed groove (37).

9. Tube and stopper combination according to claim 8, in which said first and second cylindrical portions are separated by an annular groove in the skirt.

10. Tube and stopper combination according to claim 2, in which the tube comprises an outwardly-protruding annular bead (2'') on the end of its neck (1''a) and said annular inwardly-facing groove (45) in the stopper is configured in cross-section to receive therein said annular bead (2'') on the neck of the tube to provide a sealing fit.

11. Tube and stopper combination according to claim 10, in which the stopper skirt comprises a second annular groove (46) intersecting said at least one axially-directed groove (47, 47'), said at least one axially-directed groove having a deeper section than said second annular groove (46) at their intersection, said second annular groove (46) being configured to receive said bead (2'') to hold the stopper in the venting position.

12. Tube and stopper combination according to claim 10, in which said at least one axially-directed groove (47, 47') intersects at least one further circumferential annular groove (45') in the stopper skirt (43), the axially-directed groove(s) (47, 47') having a shallower section than said further annular groove (45') at their intersection.

13. Tube and stopper combination according to claim 10, in which the stopper comprises first (45), second (45') and third (46) annular grooves spaced apart from one another in the direction from the head to the edge of the skirt and configured in cross-section to receive therein said annular bead (2'') on the neck of the tube to hold the stopper respectively in a first sealing position, a second venting position and a third sealing position, said at least one axially-directed groove (47, 47') intersecting the second (45') and third (46) annular grooves and having, at the respective intersections, a section deeper than the second annular groove (45') and a section shallower than the third annular groove (46).

14. Tube and stopper combination according to claim 10, in which the tube neck (1''') has a cylindrical inner surface extended at the open end by an outwardly flaring surface (3) of generally trunco-conical shape which terminates adjacent an outwardly-protruding curved external surface (4) of said bead (2'''), said plug (48) of the stopper being dimensioned so that when the stopper is in the sealing position with said bead engaged in said annular groove (45), the plug (48) sealably engages the cylindrical inner surface of the tube neck, the plug being shaped so that it remains out of contact with said curved external surface of said rim when the stopper is moved into and out of the sealing position.

15. Tube and stopper combination according to claim 1, in which the neck of the tube is cylindrical and extends from a generally cylindrical body of larger diameter than the neck, the skirt of the stopper having a length and outer diameter in relation to the neck and body of the tube whereby the skirt is substantially flush with said body of the tube when the stopper is fully pushed on the neck.

16. Tube and stopper combination according to claim 1, 5, 8, 11, 12 or 13, in which said at least one axially-directed groove (47) of the stopper contains a gas-permeable asepticized filtering material.

17. The stopper of the tube and stopper combination of any preceding claim.

**Revendications**

1. Une combinaison d'un tube genre fiole et d'un bouchon, le tube comprenant un col (1') ayant une extrémité ouverte et le bouchon comprenant un corps creux généralement cylindrique en matière déformable avant une tête (31) comprenant une membrane d'étanchéité (32) pour s'ajuster sur et fermer l'extrémité ouverte du tube et une jupe intégrale (33) s'étendant depuis la tête (31) pour s'ajuster sur le col (1') du tube, et au moins une rainure axiale (37) dans le bouchon s'étendant depuis le bord de la jupe au moins partiellement le long de la surface interne de ladite jupe, le bouchon étant déplaçable vers l'extérieur sur le tube depuis une position de fermeture étanche jusqu'à une position de ventilation dans laquelle ladite rainure axiale fait communiquer l'intérieur du tube avec l'extérieur, caractérisée en ce que le bouchon est muni d'une creusure annulaire (35) entourant une saillie centrale (38) formée sur la membrane d'étanchéité (32) et qui s'ajuste hermétiquement dans l'extrémité ouverte du col du tube quand le bouchon est dans la position de fermeture, et en ce que la jupe a une portion d'obturation généralement cylindrique (33'a) qui s'étend depuis ladite creusure annulaire (35) au-delà de la saillie (38) et est lisse et ininterrompue jusqu'à ce qu'elle atteigne ladite rainure axiale (37), ladite portion d'obturation (33'a) ayant un diamètre inférieur au diamètre maximum de la creusure annulaire (35) tel que dans la position de fermeture elle s'ajuste hermétiquement autour de la paroi extérieure du col du tube alors que simultanément la saillie (38) s'ajuste hermétiquement contre la paroi interne du col du tube.

2. Combinaison d'un tube et d'un bouchon selon la revendication 1, dans laquelle ladite creusure annulaire (35) a la forme d'une gorge dirigée vers l'intérieur formée dans la partie d'extrémité de la jupe, ladite gorge s'étendant au-delà de la saillie (38).

3. Combinaison d'un tube et d'un bouchon selon la revendication 2, dans laquelle le diamètre de la saillie (38) du bouchon est supérieur ou égal au diamètre de la portion d'obturation (33'a) de la jupe quand la jupe est détendue avant l'introduction du col du tube.

4. Combinaison d'un tube et d'un bouchon selon la revendication 2, dans laquelle le bouchon (41, 43) comprend au moins une autre gorge annulaire (46) dans la surface interne de la jupe (43).

5. Combinaison d'un tube et d'un bouchon selon la revendication 4, dans laquelle la ou au moins une desdites autres gorges annulaires coupe ladite rainure axiale (47, 47') de sorte que lorsque le bouchon est tiré depuis la position de fermeture étanche jusqu'à l'extrémité ouverte du col du tube engagée dans ladite gorge annulaire, le bouchon est tenu dans ladite position de ventilation.

6. Combinaison d'un tube et d'un bouchon selon la revendication 1, dans laquelle le col du tube a une surface extérieure cylindrique lisse et un bord (2') dépassant vers l'intérieur de l'extrémité ouverte du col, la saillie du bouchon étant munie sur sa surface périphérique d'une gorge annulaire (38') dirigée vers l'extérieur dont le profil est prévu pour recevoir hermétiquement ledit bord du col du tube dépassant vers l'intérieur quand le bouchon est dans la position de fermeture.

7. Combinaison d'un tube et d'un bouchon selon la revendication 1, dans laquelle ladite surface interne de la jupe s'étend vers le haut au-delà de la portion d'obturation suivant une section généralement conique (35'') s'évasant vers l'extérieur depuis ladite portion d'obturation cylindrique (33'a) jusqu'à un diamètre maximum (35') à un endroit faisant face à la paroi périphérique de la saillie.

8. Combinaison d'un tube et d'un bouchon selon la revendication 1, dans laquelle la surface interne de la jupe comprend une première et une seconde portion cylindrique, une première portion d'obturation généralement ininterrompue (33'a) adjacente à la tête du bouchon, ladite première portion d'obturation étant au plus interrompue seulement partiellement par l'extrémité de ladite rainure axiale (37) et une seconde portion cylindrique (33'b) de diamètre supérieur à celui de ladite première portion, ladite seconde portion s'étendant depuis ladite première portion adjacente jusqu'au bord de la jupe et étant interrompue par ladite rainure axiale (37).

9. Combinaison d'un tube et d'un bouchon selon la revendication 8, dans laquelle lesdites première et seconde portions cylindriques sont séparées par une gorge annulaire dans la jupe.

10. Combinaison d'un tube et d'un bouchon selon la revendication 2 dans laquelle le tube comprend un bourrelet annulaire (2'') dépassant vers l'extérieur sur l'extrémité de son col (1''a) et ladite gorge annulaire dirigée vers l'intérieur (45) formée dans le bouchon a un profil prévu pour recevoir ledit bourrelet annulaire (2'') sur le col du tube pour former un ajustement étanche.

11. Combinaison d'un tube et d'un bouchon selon la revendication 10 dans laquelle la jupe du bouchon comprend une seconde gorge annulaire (46) coupant ladite au moins une rainure axiale (47, 47'), ladite au moins une rainure axiale ayant une section plus profonde que ladite deuxième gorge annulaire (46) à leur intersection, ladite seconde gorge annulaire (46) étant prévue pour recevoir ledit bourrelet (2'') pour retenir le bouchon dans la position de ventilation.

12. Combinaison d'un tube et d'un bouchon selon la revendication 10, dans laquelle ladite au moins une rainure axiale (47, 47') coupe au moins une autre gorge circonférentielle annulaire (45') dans la jupe (43) du bouchon, la (les) rainure(s) axiale(s) (47, 47') ayant une section inférieure à cette autre gorge annulaire (45') à leur intersection.

13. Combinaison d'un tube et d'un bouchon selon la revendication 10, dans laquelle le bouchon comprend une première (45), deuxième (45') et troisième (46) gorges annulaires espacées les unes des autres depuis la tête vers le bord de la jupe et dont le profil est prévu pour recevoir ledit bourrelet annulaire (2'') sur le col du tube pour tenir le bouchon respectivement dans une première position de fermeture hermétique, une deuxième position de ventilation et une troisième position de fermeture, ladite au moins une rainure axiale (47, 47') coupant les deuxième (45') et troisième (46) gorges annulaires et ayant, aux intersections respectives, une section plus profonde que la deuxième gorge annulaire (45') et une section moins profonde que la troisième gorge annulaire (46).

14. Combinaison d'un tube et d'un bouchon selon la revendication 10, dans laquelle le col du tube (1''') a une surface interne cylindrique prolongée à l'extrémité ouverte par une surface évasée vers l'extérieur (3) d'une forme généralement tronconique qui se termine à côté d'une surface incurvée (4) du bourrelet (2''') faisant saillie vers l'extérieur, ladite saillie (48) du bouchon ayant une dimension telle que lorsque le bouchon est dans la position de fermeture étanche avec ledit bourrelet engagé dans ladite gorge annulaire (45), la saillie (48) engage hermétiquement la face interne cylindrique du col du tube, la saillie étant formée de telle sorte qu'elle reste hors de contact avec ladite surface externe incurvée dudit bord quand le bouchon est déplacé dans et hors de sa position de fermeture.

15. Combinaison d'un tube et d'un bouchon selon la revendication 1, dans lequel le col du tube est cylindrique et s'étend depuis un corps généralement cylindrique d'un diamètre supérieur à celui du col, la jupe du bouchon ayant une longueur et un diamètre extérieur en relation avec le col et le corps du tube de telle sorte que la jupe est substantiellement de niveau avec ledit corps du tube quand le bouchon est complètement poussé sur le col.

16. Combinaison d'un tube et d'un bouchon selon les revendications 1, 5, 8, 11, 12 ou 13 dans laquelle ladite au moins une rainure axiale (47) du bouchon contient une matière filtrante perméable aux gaz et aseptisée.

17. Bouchon du tube et combinaison de bouchon selon l'une quelconque des revendications précédentes.

**Patentansprüche**

1. Kombination einer ampullenartigen Röhre mit einem offenen Hals (1') und eines hohlzylindrischen Stöpsels aus verformbarem Material mit einem eine Dichtungsmembran (32) aufweisenden Kopfstück (31) zum Aufsetzen und Verschliessen des offenen Röhrenendes und mit einem an dem Kopfstück (31) sitzenden Mantel (33) zum Aufziehen auf den Röhrenhals (1') sowie mit wenigstens einer axial verlaufenden Nut (37) in dem Stöpsel, welche vom Mantelrand aus wenigstens teilweise entlang der Innenfläche dieses Mantels verläuft, wobei der Stöpsel auf der Röhre von einer Abdichtungsstellung nach aussen in eine Belüftungsstellung ziehbar ist, in welcher die Axialnut das Röhreninnere mit aussen verbindet, dadurch gekennzeichnet, dass in dem Stöpsel eine ringförmige Ausnehmung (35) um einen herausstehenden Mittelkern (38) an der Dichtungsmembran (32) verläuft, welcher dicht in dem offenen Röhrenende sitzt, wenn der Stöpsel sich in seiner Abdichtungsstellung befindet, und dass der Mantel einen etwa zylindrischen Dichtungsabschnitt (33'a) aufweist, welcher von der ringförmigen Ausnehmung (35) um den Kern (38) ausgeht und glatt und ununterbrochen bis zu der Axialnut (37) reicht und welcher einen geringeren Durchmesser aufweist als der maximale Aussendurchmesser der ringförmigen Ausnehmung (35), so dass dieser Mantel in der Abdichtungsstellung die Aussenwand des Röhrenhalses dicht umgibt, wenn gleichzeitig der Kern (38) dicht an der Innenwand des Röhrenhalses sitzt.

2. Röhren- und Stöpselkombination nach Anspruch 1, dadurch gekennzeichnet, dass die ringförmige Ausnehmung (35) die Form einer nach innen offenen Nut am Endteil des Mantels aufweist, welche sich bis über den Kern (38) erstreckt.

3. Röhren- und Stöpselkombination nach Anspruch 2, dadurch gekennzeichnet, dass der Durchmesser des Stöpselkernes (38) grösser als der oder gleich dem Durchmesser des Dichtungsabschnittes (33'a) des Mantels ist, wenn der Mantel vor dem Aufziehen auf den Röhrenhals ungedehnt ist.

4. Röhren- und Stöpselkombination nach Anspruch 2, dadurch gekennzeichnet, dass der Stöpsel (41, 43) wenigstens eine weitere umlaufende Ringnut (46) an der Innenfläche des Mantels (43) aufweist.

5. Röhren- und Stöpselkombination nach Anspruch 4, dadurch gekennzeichnet, dass wenigstens eine der Ringnuten die Axialnut (47, 47') schneidet, so dass beim Herausziehen des Stöpsels aus seiner Abdichtungsstellung, bis das obere Ende des Röhrenhalses in diese Ringnut eingreift, der Stöpsel in dieser Belüftungsstellung gehalten wird.

6. Röhren- und Stöpselkombination nach Anspruch 1, dadurch gekennzeichnet, dass der Röhrenhals eine glatte zylindrische Aussenfläche hat und eine Rippe (2') am offenen Ende des Halses nach innen hervorsteht und dass der Stöpselkern an seiner Umfangsfläche eine nach aussen offene Nut (38') mit einem solchen Querschnitt aufweist, dass sie die nach innen hervorstehende Rippe am Röhrenhals aufnimmt, wenn sich der Stöpsel in seiner Abdichtungsstellung befindet.

7. Röhren- und Stöpselkombination nach Anspruch 1, dadurch gekennzeichnet, dass die Innenfläche des Mantels sich nach oben über dem Dichtungsabschnitt als ein konischer Abschnitt (35') erstreckt, welcher sich von dem zylindrischen Dichtungsabschnitt (33'a) bis auf einen maximalen Durchmesser (35') an einer Stelle gegenüber der Umfangswand des Kernes nach aussen erweitert.

8. Röhren- und Stöpselkombination nach Anspruch 1, dadurch gekennzeichnet, dass die Innenfläche des Mantels aus zwei zylindrischen Abschnitten besteht, von denen der eine der ununterbrochene Dichtungsabschnitt (33'a) nahe dem Stöpselkopfstück ist und höchstens nur teilweise von einem Ende der Axialnut (37) unterbrochen wird, während der andere zylindrische Abschnitt (33'b) einen grösseren Durchmesser als der erste Abschnitt aufweist und sich von hier bis zum Mantelrand erstreckt und von der Axialnut (37) unterbrochen ist.

9. Röhren- und Stöpselkombination nach Anspruch 8, dadurch gekennzeichnet, dass die beiden zylindrischen Abschnitte durch eine Ringnut in dem Mantel getrennt sind.

10. Röhren- und Stöpselkombination nach Anspruch 2, dadurch gekennzeichnet, dass die Röhre mit einer nach aussen hervorstehenden Ringrippe (2'') am Ende ihres Halses (1''a) versehen ist und dass die nach innen offene Ringnut (45) in dem Stöpsel einen solchen Querschnitt aufweist, dass sie diese Ringrippe (2'') am Röhrenhals unter dichtem Sitz aufnimmt.

11. Röhren- und Stöpselkombination nach Anspruch 10, dadurch gekennzeichnet, dass der Mantel eine zweite Ringnut (46) aufweist, welche wenigstens eine Axialnut (47, 47') mit einem tieferen Querschnitt am Schnittpunkt als die Ringnut (46) schneidet und welche so geformt ist, dass sie die Ringrippe (2'') aufnimmt und den Stöpsel in seiner Belüftungsstellung hält.

12. Röhren- und Stöpselkombination nach Anspruch 10, dadurch gekennzeichnet, dass wenigstens eine Axialnut (47, 47') wenigstens eine weitere umlaufende Ringnut (45') in dem Stöpselmantel (34) schneidet und dass diese Axialnut (47, 47') am Schnittpunkt einen flacheren Querschnitt als die weitere Ringnut (45') aufweist.

13. Röhren- und Stöpselkombination nach Anspruch 10, dadurch gekennzeichnet, dass der Stöpsel drei getrennt voneinander zwischen dem Kopfstück und dem Mantelrand angeordnete Ringnuten (45, 45', 46) mit einem solchen Querschnitt aufweist, dass sie die Ringrippe (2'') am Röhrenhals aufnehmen und den Stöpsel jeweils in einer ersten Abdichtungsstellung, in einer zweiten Belüftungsstellung und in einer dritten Abdichtungsstellung halten, und dass wenigstens eine Axialnut (47, 47') die zweite Ringnut (45') und die dritte Ringnut (46) schneidet und an den jeweiligen Schnittpunkten einen tieferen Querschnitt als die zweite Ringnut (45') und einen flacheren Querschnitt als die dritte Ringnut (46) aufweist.

14. Röhren- und Stöpselkombination nach Anspruch 10, dadurch gekennzeichnet, dass der Röhrenhals (1''') eine zylindrische Innenfläche mit einem sich am offenen Ende stumpfkonisch erweiternden Abschnitt (3) aufweist, welcher nahe einer nach aussen heraustehenden, gekrümmten Aussenfläche (4) der Ringrippe (2''') endet, und dass der Kern (48) des Stöpsels solche Abmessungen aufweist, dass in der Abdichtungsstellung, wobei die Ringrippe in der Ringnut (45) sitzt, der Kern (48) abdichtend an der zylindrischen Innenfläche des Röhrenhalses anliegt, und so geformt ist, dass er ausser Berührung mit der gekrümmten Aussenfläche der Rippe bleibt, wenn der Stöpsel in die und aus der Abdichtungsstellung gebracht wird.

15. Röhren- und Stöpselkombination nach Anspruch 1, dadurch gekennzeichnet, dass der Röhrenhals zylindrisch ist und von einem ebenfalls zylindrischen Körper mit grösserem Durchmesser als der Hals ausgeht und dass der Mantel des Stöpsels eine Länge und einen Aussendurchmesser entsprechend dem Hals und dem Körper aufweist, indem der Mantel in gleicher Radialhöhe wie der Röhrenkörper liegt, wenn der Stöpsel vollständig auf den Hals aufgeschoben ist.

16. Röhren- und Stöpselkombination nach Anspruch 1, 5, 8, 11, 12 oder 13, dadurch gekennzeichnet, dass wenigstens eine Axialnut (47) des Stöpsels ein gasdurchlässiges, aseptisches Filtermaterial enthält.

17. Der Röhrenstöpsel und die Stöpselkombination eines vorhergehenden Anspruches.

Fig.1

Fig.3

Fig.2

Fig. 4

Fig. 5

Fig. 7

Fig. 6

Fig. 8

Fig. 9